# EUROPÄISCHE PATENTANMELDUNG

(11) **EP 2 080 529 A1**
(43) Veröffentlichungstag der Anmeldung: **22.07.2009**
(21) Anmeldenummer: 08100641.3
(22) Anmeldetag: 18.01.2008
(51) Int. Cl.: A61M 1/00, A61M 1/36

(54) **Absaugsystem und Verfahren für das Absaugen von Flüssigkeiten bei chirurgischen Eingriffen**

(71) Anmelder: Cardiosmart AG, 5630 Muri (CH)
(72) Erfinder: Christen, Urs, 5637, Geltwil (CH)
(74) Vertreter: Scheuzger, Beat Otto

(57) **Zusammenfassung**

Die Erfindung betrifft ein Absaugsystem für das Absaugen von Flüssigkeiten aus einem Operationsgebiet (9). Ein Sauger (112) ist über eine Absaugleitung (11) mit einem ersten Reservoir (1) verbunden. Das erste Reservoir (1) ist über eine erste Unterdruckleitung (121) mit einer ersten Unterdruckeinheit (12) verbunden. Das erste Reservoir (1) ist über eine Ventileinrichtung (16) mit einem zweiten Reservoir (2) verbunden, wobei das zweite Reservoir (2) über eine zweite Unterdruckleitung (221) mit einer zweiten Unterdruckeinheit (22) verbunden ist, und wobei mittels der Ventileinrichtung (16) die Verbindung zwischen dem ersten Reservoir (1) und dem zweiten Reservoir (2) geöffnet oder geschlossen werden kann.

## Beschreibung

### Technisches Gebiet

Die vorliegende Erfindung bezieht sich auf ein Absaugsystem und ein Verfahren zum Absaugen von Flüssigkeiten bei chirurgischen Eingriffen. Das Absaugsystem umfasst insbesondere einen Sauger, welcher über eine Absaugleitung mit einem ersten Reservoir verbunden ist, wobei das erste Reservoir über eine erste Unterdruckleitung mit einer ersten Unterdruckeinheit verbunden ist.

### Stand der Technik

Im Stand der Technik sind Absaugvorrichtungen zum Absaugen von Flüssigkeiten bei chirurgischen Eingriffen bekannt. So beschreibt beispielsweise die Patentschrift EP 1764121 eine Absaugvorrichtung für das Absaugen von Flüssigkeiten aus einem Operationsgebiet, welche einen Sauger umfasst, wobei an der Saugerspitze ein optischer Sensor angebracht ist, mittels welchem detektierbar ist, ob sich die Saugerspitze in der Flüssigkeit befindet oder in der Luft befindet. Der Sauger wird in einen Saugmodus geschaltet, falls sich die Saugerspitze in der Flüssigkeit befindet und der Saugmodus wird ausgeschaltet, sobald sich der Sauger in der Luft befindet. Mit dieser Vorrichtung kann es vermieden werden, dass die Flüssigkeit durch das Ansaugen von Luft aufgeschäumt wird und durch das Aufschäumen die Qualität der abgesaugten Flüssigkeit beeinträchtigt wird. Bei der abgesaugten Flüssigkeit handelt es sich beispielsweise um Blut, welches in einem Operationsgebiet durch chirurgische Eingriffe aus Wunden des Operationsgebiets austritt. So kann bei einer Herzoperation durch eine solche Absaugvorrichtung erreicht werden, dass das Operationsgebiet nicht durch Blut verdeckt ist und dass das abgesaugte Blut in einer guten Qualität, d.h. insbesondere ohne Schaumbildung, in einem Reservoir gesammelt werden kann. Bei einer Herzoperation, welche beispielsweise 90 bis 120 Minuten dauert, können mit einer solchen Absaugvorrichtung zwischen 4 bis 6 Liter Blut aus dem Operationsgebiet abgesaugt werden. Dabei kann das Blut in einem Reservoir gesammelt werden und, da es in einer guten Qualität im Reservoir vorliegt, dem Blutkreislauf des Patienten wieder zugeführt werden. Um eine Absaugwirkung zu erzielen, kann das Reservoir an eine Unterdruckeinheit angeschlossen sein und der Sauger kann über einen Schlauch mit dem Reservoir verbunden sein, wobei der Schlauch mittels einer Schliessvorrichtung, welche mittels des optischen Sensors steuerbar ist, zusammengedrückt werden kann. Durch eine solche Absaugvorrichtung wird ein schonendes Absaugen von Blut aus einem Operationsgebiet bewirkt. Um das im Reservoir gesammelte Blut in den Blutkreislauf zurückzuführen muss das Reservoir entleert werden. Dazu muss zuerst der Unterdruck im Reservoir abgebaut werden, worauf eine Entleerung des Reservoirs erfolgen kann. Da bei einem solchen Entleervorgang im Reservoir kein Unterdruck besteht, kann während dem Entleervorgang allerdings kein Blut abgesaugt werden. Es gibt allerdings Operationen (??Beispiele), bei welchen während der Dauer der Operation viel grössere Mengen an Blut aus einem Operationsgebiet abgesaugt werden muss. So kann es sein, dass während einer solchen Operation bis zu 50 oder 60 Liter Blut abgesaugt werden. Dabei ist es nicht nur sehr wichtig, das Blut besonders schonend abzusaugen, sondern es ist auch wichtig, das Blut möglichst kontinuierlich wieder in den Blutkreislauf des Patienten zurückzuführen. Im Stand der Technik ist jedoch eine solche kontinuierliche Rückführung von Blut in den Blutkreislauf eines Patienten nicht möglich.

### Darstellung der Erfindung

Es ist eine Aufgabe der vorliegenden Erfindung, ein neues Absaugsystem und ein Verfahren zum Absaugen von Flüssigkeiten aus einem Operationsgebiet vorzuschlagen, welche nicht die Nachteile des Standes der Technik aufweisen. Das neue Absaugsystem und das neue Verfahren zum Absaugen von Flüssigkeiten sollen insbesondere bewirken, dass abgesaugte Flüssigkeit kontinuierlich in einen Flüssigkeitskreislauf zurückgeführt werden kann. Zum Absaugen von Flüssigkeiten umfasst das erfindungsgemässe Absaugsystem einen Sauger, welcher über eine Absaugleitung mit einem ersten Reservoir verbunden ist. Das erste Reservoir ist über eine erste Unterdruckleitung mit einer ersten Unterdruckeinheit verbunden.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Insbesondere werden diese Ziele durch die Erfindung dadurch erreicht, dass das erste Reservoir über eine Ventileinrichtung mit einem zweiten Reservoir verbunden ist, wobei das zweite Reservoir über eine zweite Unterdruckleitung mit einer zweiten Unterdruckeinheit verbunden ist, und wobei mittels der Ventileinrichtung die Verbindung zwischen dem ersten Reservoir und dem zweiten Reservoir geöffnet oder geschlossen werden kann. Ein solches Absaugsystem hat insbesondere den Vorteil, dass der Sauger ununterbrochen für das Absaugen von Flüssigkeiten aus einem Operationsgebiet verwendet werden kann und dass abgesaugte Flüssigkeit bei Bedarf einen bestehenden Flüssigkeitskreislauf zugeführt werden kann.

In einer Ausführungsvariante ist das zweite Reservoir mit einer weiteren Absaugleitung verbunden, wobei die weitere Absaugleitung zum Absaugen von Flüssigkeiten aus einem Operationsgebiet verwendbar ist. Ein solches Absaugsystem hat insbesondere den Vorteil, dass mit einer weiteren Absaugleitung zusätzliche Absaugleistung von Flüssigkeiten aus einem Operationsgebiet zur Verfügung gestellt werden kann.

In einer weiteren Ausführungsvariante umfassen die erste Unterdruckeinheit und/oder die zweite Unterdruckeinheit mindestens eine Einstellvorrichtung zur Einstellung eines selektierbaren Unterdrucks. Ein solches Absaugsystem hat insbesondere den Vorteil, dass die Absaugleistung des Absaugsystems an einen bestimmten Bedarf angepasst werden kann.

In einer anderen Ausführungsvariante umfassen das erste Reservoir und/oder des zweite Reservoir mindestens eine Filtriereinrichtung zur Filtrierung von abgesaugten Flüssigkeiten. Eine solche Ausführungsvariante hat insbesondere den Vorteil, dass die abgesaugte Flüssigkeit einfach aufgearbeitet werden kann und schnell in einer erwünschten Qualität zur Verfügung gestellt werden kann.

In einer weiteren Ausführungsvariante ist das zweite Reservoir über eine Ablaufeinrichtung mit einem Flüssigkeitsspeicher verbunden. Ein solches Absaugsystem hat insbesondere den Vorteil, dass abgesaugte Flüssigkeit direkt in einem bestehenden Flüssigkeitsspeicher zugeführt werden kann.

In einer anderen Ausführungsvariante wird der Flüssigkeitsspeicher durch eine Herz-Lungen-Maschine gebildet. Ein solches Absaugsystem hat insbesondere den Vorteil, dass bei Operationen abgesaugtes Blut direkt dem Blutkreislauf zurückgeführt werden kann.

Bei einem Verfahren zum Absaugen von Flüssigkeiten aus einem Operationsgebiet kann die Ventileinrichtung zwischen dem ersten Reservoir und dem zweiten Reservoir verschlossen werden und es kann mittels der ersten Unterdruckeinheit ein erster Unterdruck im ersten Reservoir eingestellt werden, wobei mittels der zweiten Unterdruckeinheit ein zweiter Unterdruck im zweiten Reservoir derart eingestellt wird, dass beim Öffnen der Ventileinrichtung Flüssigkeit aus dem ersten Reservoir in das zweite Reservoir transportiert wird.

### Kurze Beschreibung der Zeichnungen

Nachfolgend werden Ausführungsvarianten der vorliegenden Erfindung anhand von Beispielen beschrieben. Die Beispiele der Ausführungen werden durch folgende beigelegte Figur illustriert:
Figur 1 zeigt ein erfindungsgemässes Absaugsystem mit zwei getrennten Reservoirs.
Figur 2 zeigt ein erfindungsgemässes Absaugsystem mit einem kombinierten Reservoir.

### Wege zur Ausführung der Erfindung

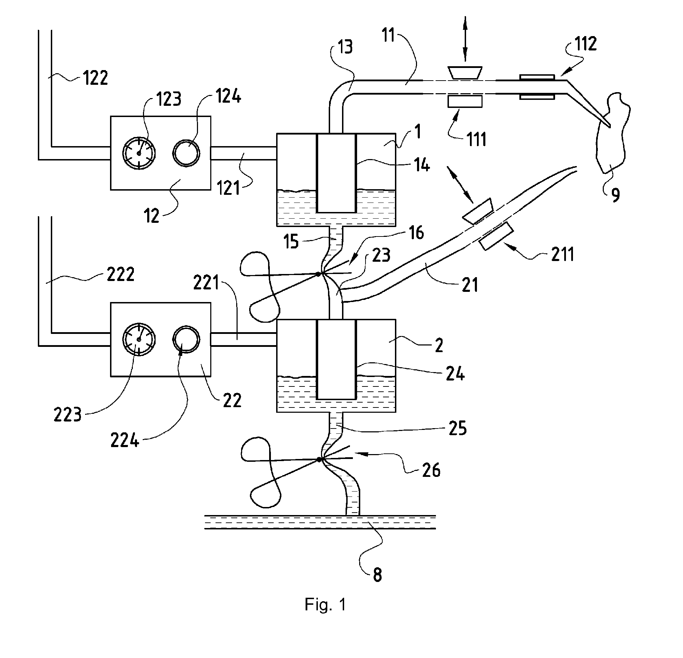

In Figur 1 bezieht sich das Bezugszeichen 1 auf ein erstes Reservoir. Das erste Reservoir 1 kann einen Eingangsbereich 13, eine Filtriereinrichtung 14 und einen Ausgangsbereich 15 umfassen. Ein solches erstes Reservoir 1 kann insbesondere aus einem durchsichtigen Kunststoff wie beispielsweise einem PVC Kunststoff, einem Polycarbonat (PC) Kunststoff, einem SAN Kunststoff, einem ABS Kunststoff oder aus irgendeinem anderen Material hergestellt sein. Der Eingangsbereich 13 und der Ausgangsbereich 15 können beispielsweise verschiedene Einlässe oder Auslässe umfassen, an welche Einlässe oder Auslässe Schläuche von normierten Grössen wie beispielsweise 3/16 Zoll Schläuche oder 1/4 Zoll Schläuche angeschlossen werden können. Die im Eingangsbereich 13 oder im Ausgangsbereich 15 angeordneten Einlässe und Auslässe können beispielsweise drehbar eingerichtet sein. In Figur 1 bezieht sich das Bezugszeichen 112 auf einen Sauger. Der Sauger 112 kann eine Spitze umfassen, welche zum Absaugen von Flüssigkeiten in die entsprechende Flüssigkeit getaucht wird. Der Sauger 112 kann einen Griff umfassen, damit der Sauger 112 zuverlässig in der Hand eines Benutzers des Saugers gehalten werden kann. Wie in Figur 1 skizziert, kann der Sauger 112 über einen Schlauch 11 und über den Eingangsbereich 13 mit dem ersten Reservoirs 1 verbunden sein. Zwischen dem Sauger 112 und dem Eingangsbereich 13 kann am Schlauch 11 eine Schliessvorrichtung 111 angebracht sein, um den Schlauch 11 zu verschliessen. Die Schliessvorrichtung 111 kann wie in der Patentschrift EP 1764121 beschrieben mittels eines optischen Sensors so gesteuert werden, dass die Schliessvorrichtung 111 geschlossen ist, falls sich der Sauger 112 in der Luft befindet und dass die Schliessvorrichtung 111 geöffnet ist, falls sich der Sauger 112 in einer Flüssigkeit wie Blut befindet. Mit einer derart steuerbaren Schliessvorrichtung 111 kann das Schäumen von abgesaugter Flüssigkeit, beispielsweise also das Schäumen von abgesaugtem Blut, stark reduziert werden.

In Figur 1 bezieht sich das Bezugszeichen 12 auf eine erste Unterdruckeinheit. Die erste Unterdruckeinheit 12 kann über eine Leitung 122 an ein in einem Operationssaal standardmässig installiertes Unterdrucksystem angeschlossen sein. Wie in Figur 1 skizziert, kann die erste Unterdruckeinheit 12 eine Anzeige 123 umfassen, um einen eingestellten Unterdruck beispielsweise in einer Einheit wie Pascal, mm Hg, at oder irgendeiner anderen Einheit anzuzeigen. Die erste Unterdruckeinheit 12 kann einen Regler 124 zum einstellen eines gewünschten Unterdrucks umfassen. Die erste Unterdruckeinheit 12 kann über eine Unterdruckleitung 121 mit dem ersten Reservoir 1 verbunden sein. Der auf der Anzeige 123 angezeigte Unterdruck kann beispielsweise dem Unterdruck in der Verbindung 121 und somit dem Unterdruck im ersten Reservoir 1 entsprechen.

In Figur 1 bezieht sich das Bezugszeichen 14 auf eine Filtriereinrichtung. Die Filtriereinrichtung 14 kann aus irgendwelchen flüssigkeitsdurchlässigen Materialen bestehen, beispielsweise aus einem Kunststoffgewebe, einem Polyurethanschaum oder aus irgendeinem anderen Material. Die Filtriereinrichtung 14 kann dazu dienen, unerwünschte Bestandteile wie Knochensplitter oder irgendwelche andere Bestandteile, welche durch den Sauger 112 aus dem Operationsgebiet 9 abgesaugt werden, zurückzubehalten. Die Filtriereinrichtung 14 kann eingerichtet sein, um die abgesaugte Flüssigkeit zu Entschäumen. Die Filtriereinrichtung 14 kann weiter eingerichtet sein, um andere Funktionen oder eine Kombination von Funktionen zu erzielen. Wie in Figur 1 skizziert, kann der Eingangsbereich 13 in einen Innenbereich der Filtriereinrichtung 14 führen und die abgesaugte Flüssigkeit kann in einem Aussenbereich der Filtriereinrichtung 14 gesammelt werden. Die Verwendung der Filtriereinrichtung 14 ist optional und kann je nach Anwendung ganz entfallen.

In Figur 1 bezieht sich das Bezugszeichen 16 auf eine Ventileinrichtung, welche zwischen dem ersten Reservoir 1 und einem zweiten Reservoir 2 angeordnet ist. Die Ventileinrichtung 16 kann mittels eines Schlauchs gebildet sein, welcher vom Ausgangsbereich 15 des ersten Reservoirs 1 zu einem Eingangsbereich 23 des zweiten Reservoirs führt, und mittels einer von Hand entfernbaren Klemme gebildet sein, welche es ermöglicht den Schlauch zusammenzudrücken. Mit einem solchen Schlauch und einer solchen Klemme kann die Ventileinrichtung 16 zwischen einem geschlossenen Zustand und einem geöffneten Zustand eingestellt werden. Selbstverständlich kann statt einer solchen Ventileinrichtung irgendeine andere Ventileinrichtung verwendet werden, beispielsweise eine Ventileinrichtung welche einen mechanisch bedienbaren Schieber umfasst, eine Ventileinrichtung welche ein elektrisch bedienbares Ventil umfasst oder irgendeine andere Ventileinrichtung, mit welcher die Verbindung zwischen dem ersten Reservoir 1 und dem zweiten Reservoir 2 geöffnet respektive geschlossen werden kann. Je nach dem kann es auch sinnvoll sein, eine Ventileinrichtung zu verwenden, welche eine teilweise Öffnung der Verbindung zwischen dem ersten Reservoir 1 und dem zweiten Reservoir 2 ermöglicht. Es ist auch denkbar, eine steuerbare Ventileinrichtung 16 zu verwenden, wobei zur Steuerung der Ventileinrichtung beispielsweise der Füllstand des ersten Reservoirs 1 und/oder der Füllstand des zweiten Reservoirs 2 gemessen wird und die steuerbare Ventileinrichtung beispielsweise entsprechend dem Füllstand gesteuert wird. Selbstverständlich kann es vorgesehen sein, für eine solche steuerbare Ventileinrichtung 16 entsprechende Sensoren, wie einen Füllstandsensor, entsprechende Hardware oder Softwaremodule, wie beispielsweise einen programmierbaren Mikroprozessor, sowie entsprechende Steuereinrichtungen, wie beispielsweise ein Ventil welches durch einen Elektromotor angetrieben ist, anzuordnen.

In Figur 1 bezieht sich das Bezugszeichen 2 auf ein zweites Reservoir. Das zweite Reservoir 2 kann analog zum ersten Reservoir 1 aufgebaut sein. Das zweite Reservoir 2 kann einen Eingangsbereich 23, eine Filtriereinrichtung 24 und einen Ausgangsbereich 25 umfassen. Analog wie beim ersten Reservoir 1, kann der Eingangsbereich 23 mit einem Ende des Schlauchs 21 verbunden sein. Das andere Ende des Schlauchs 21 kann mit einem weiteren Sauger zum Absaugen von Flüssigkeiten verbunden sein. Allerdings kann das andere Ende des Schlauchs 21 beispielsweise auch mit einem Vent Katheter verbunden sein, sodass der linke Ventrikel eines Herzens bei einer Herzoperation blutleer gehalten werden kann. Selbstverständlich sind weitere Anwendungen denkbar, um das andere Ende des Schlauchs 21 zur Aufnahme von Flüssigkeiten zu nutzen. Am Schlauch 21 kann wiederum eine steuerbare Schliessvorrichtung 211 angebracht sein. Die Steuerung der Schliessvorrichtung 211 kann beispielsweise auch entsprechend einer Schalterstellung eines von einem Benutzer einstellbaren Schalters erfolgen.

In Figur 1 bezieht sich das Bezugszeichen 22 auf eine zweite Unterdruckeinheit. Die zweite Unterdruckeinheit 22 kann über eine Leitung 222 an ein in einem Operationssaal standardmässig installiertes Unterdrucksystem angeschlossen sein. Die zweite Unterdruckeinheit 22 kann analog zur ersten Unterdruckeinheit 12 aufgebaut sein. Die zweite Unterdruckeinheit 22 kann wiederum eine Anzeige 223 umfassen, um einen eingestellten Unterdruck anzuzeigen und einen Regler 124 zum einstellen eines gewünschten Unterdrucks umfassen. Die zweite Unterdruckeinheit 22 kann über eine Unterdruckleitung 221 mit dem zweiten Reservoir 2 verbunden sein. Der auf der Anzeige 223 angezeigte Unterdruck kann beispielsweise dem Unterdruck in der Unterdruckleitung 221 und somit dem Unterdruck im zweiten Reservoir 2 entsprechen.

Mittels der ersten Unterdruckeinheit 12 kann im ersten Reservoir 1 ein erster Unterdruck eingestellt werden und mittels der zweiten Unterdruckeinheit 22 kann im zweiten Reservoir 2 ein zweiter Unterdruck eingestellt werden. Der Wert des ersten Unterdrucks kann sich vom Wert des zweiten Unterdrucks unterscheiden. So kann beispielsweise im ersten Reservoir 1 ein Unterdruck von 100 mm Hg erstellt werden und im zweiten Reservoir 2 kann beispielsweise ein Unterdruck von 80 mm Hg erstellt werden. Bei einer Cell Saver Applikation kann der Unterdruck im Reservoir 1 beispielsweise 0-100 mmHg und im Reservoir 2 beispielsweise 0-150 mmHg sein. Bei einer MECC Applikation kann der Unterdruck im Reservoir 2 beispielsweise 0-80 mmHg sein. Bei einer Vent Applikation kann der Unterdruck im Reservoir 1 beispielsweise 0-40 mmHg sein. Diese Werte werden hier nur als Beispiel genannt und es können selbstverständlich ganz andere Werte für den Unterdruck im Reservoir 1 und dem Unterdruck im Reservoir 2 gewählt werden. Durch den Unterschied im Unterdruck des ersten Reservoirs 1 und des zweiten Reservoirs 2 kann erreicht werden, dass beim Öffnen der Ventilvorrichtung 16 insbesondere Flüssigkeiten, aber beispielsweise auch Gase, welche sich im ersten Reservoir 1 befinden, in das zweite Reservoir 2 transportiert werden. Dabei kann das Absaugen von Flüssigkeiten, welches mittels des Schlauchs 11 und mittels des Schlauchs 21 erfolgt, vollumfänglich beibehalten werden.

In Figur 1 bezieht sich das Bezugszeichen 8 auf einen Flüssigkeitskreislauf. Ein solcher Flüssigkeitskreislauf kann insbesondere durch einen Blutkreislauf einer Herz-Lungen- Maschine gebildet werden. Dabei ist ein Ausgang der Herz-Lungen-Maschine mit der Aorta eines Patienten verbunden und ein Eingang der Herz-Lungen-Maschine ist mit der Vene des Patienten verbunden. Durch eine Pumpe und einen Oxygenator der Herz-Lungen-Maschine kann die Blutzirkulation eines Patienten auch bei stillgelegtem Herzen sichergestellt werden.

Wie in Figur 1 skizziert, kann ein Ausgang 25 des Reservoirs 2 über eine weitere Ventileinrichtung 26 mit dem Flüssigkeitskreislauf 8 verbunden sein. So kann beispielsweise die Ventileinrichtung 16 geschlossen werden und es kann mittels der zweiten Unterdruckeinheit 22 im Reservoir 2 ein Druck derart eingestellt werden, dass beim Öffnen der weiteren Ventileinrichtung 26 die im Reservoir 2 gesammelte Flüssigkeit in den Flüssigkeitskreislauf 8 übergeführt wird. Dabei kann das Absaugen von Flüssigkeiten, welches mittels des Schlauchs 11 erfolgt, vollumfänglich beibehalten werden.

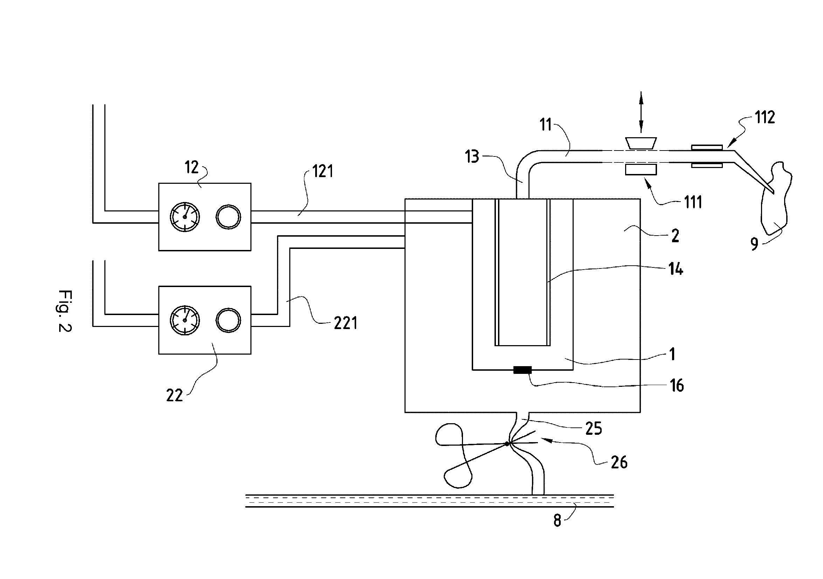

In Figur 2 ist ein kombiniertes Reservoir skizziert. Das kombinierte Reservoir kann ein erstes Reservoir 1 und ein zweites Reservoir 2 umfassen, Das erste Reservoir 1 kann innerhalb des zweiten Reservoirs 2 angeordnet sein. Im ersten Reservoir kann eine Filtriereinrichtung 14 angeordnet sein. Das erste Reservoir 1 kann über einen Eingangsbereich 13 mit einem Ende eines Schlauchs 11 verbunden sein. Am anderen Ende des Schlauchs 11 kann ein Sauger 112 zum Absaugen von Flüssigkeiten aus einem Operationsgebiet 9 angeordnet sein. Am Schlauch 11 kann eine Schliessvorrichtung zum Verschliessen des Schlauchs 11 angeordnet sein. Die Schliessvorrichtung 111 kann über irgendwelche Steuersignale, beispielsweise über Steuersignale, welche aufgrund eines am Sauger 112 angebrachten Lichtleiters zum optischen Erkennen des Umgebungsmediums an der Spitze des Saugers 112 erzeugt werden, gesteuert werden. Mittels einer derart gesteuerten Schliessvorrichtung 111 kann insbesondere erreicht werden, dass die Schliessvorrichtung 111 geschlossen ist, falls sich die Spitze des Saugers 112 in der Luft befindet und dass die Schliessvorrichtung 111 geöffnet ist, falls sich die Spitze des Saugers 112 in einer Flüssigkeit befindet.

Es ist selbstverständlich möglich, dass auch im zweiten Reservoir 2 eine Filtriereinrichtung angeordnet ist und dass ein weiterer Schlauch zum Absaugen von Flüssigkeiten mit dem zweiten Reservoir 2 entsprechend verbunden ist.

Wie in Figur 2 skizziert, kann das erste Reservoir 1 über eine erste Unterdruckleitung 121 mit einer ersten Unterdruckeinheit 12 verbunden sein. Ebenfalls kann das zweite Reservoir 2 über eine zweite Unterdruckleitung 221 mit einer zweiten Unterdruckeinheit 22 verbunden sein. Beispielsweise mittels eines Reglers und einer Anzeige kann mittels der ersten Unterdruckeinheit 12 und der zweiten Unterdruckeinheit 22 im ersten Reservoir 1 und im zweiten Reservoir 2 ein bestimmter Unterdruck eingestellt werden.

In Figur 2 bezieht sich das Bezugszeichen 16 auf eine Ventileinrichtung, welche zwischen dem ersten Reservoir 1 und dem zweiten Reservoir 2 angeordnet ist. Die Ventileinrichtung 16 kann beispielsweise einen Schieber umfassen, welcher über eine Schiebevorrichtung (in der Figur nicht skizziert) bedienbar ist. Der Schieber kann so angeordnet sein, dass eine Öffnung zwischen dem ersten Reservoir 1 und dem zweiten Reservoir 2 geöffnet oder verschlossen werden kann. Selbstverständlich kann die Ventileinrichtung 16 auch elektromechanische Bauteile umfassen, sodass eine Öffnung zwischen dem ersten Reservoir 1 und dem zweiten Reservoir 2 mittels elektromechanischen Einrichtungen, wie beispielsweise einem Magnetventil, geöffnet oder verschlossen werden kann. Selbstverständlich kann die Ventileinrichtung 16 so eingerichtet sein, dass eine Öffnung zwischen dem ersten Reservoir 1 und dem zweiten Reservoir 2 auch nur teilweise geöffnet oder verschlossen werden kann.

In Figur 2 bezieht sich das Bezugszeichen 25 auf einen Ausgang des zweiten Reservoirs 2. Wie in Figur 2 skizziert kann der Ausgang 25 des zweiten Reservoirs über eine weitere Ventileinrichtung 26 mit einem Flüssigkeitskreislauf 8 verbunden sein.

Bei der Verwendung des kombinierten Reservoirs kann mittels der ersten Unterdruckeinheit 12 im ersten Reservoir 1 ein Unterdruck so eingestellt werden, dass mit dem Sauger 112 Flüssigkeiten aus einem Operationsgebiet 9 mit einer gewünschten Leistung abgesogen werden kann. Dabei können die Ventileinrichtung 16 und die weitere Ventileinrichtung 26 geschlossen sein. Mittels der Unterdruckeinheit 22 kann im zweiten Reservoir 2 ein Unterdruck so eingestellt werden, dass Flüssigkeit bei geöffneter Ventileinrichtung 16 vom ersten Reservoir 1 in das zweite Reservoir 2 transportiert wird. So kann beispielsweise bei einem gewissen Füllstand des Reservoirs 1 die Ventileinrichtung 16 geöffnet werden und das erste Reservoir 1 kann in das zweite Reservoir 2 entleert werden. Dabei steht am Sauger 112 stets die volle Saugleistung zur Verfügung. Nachdem die Ventileinrichtung 16 geschlossen ist, kann beispielsweise mittels der Unterdruckeinheit 22 der Unterdruck im zweiten Reservoir 2 so eingestellt werden, dass bei geöffneter weiterer Ventileinrichtung 26 Flüssigkeit vom zweiten Reservoir 2 in den Flüssigkeitskreislauf 8 transportiert wird. So kann bei einem gewissen Füllstand des zweiten Reservoirs 2 die Ventileinrichtung 26 geöffnet werden und das zweite Reservoir 2 kann in den Flüssigkeitskreislauf 8 entleert werden.

Selbstverständlich kann die Entleerung des zweiten Reservoirs 2 auch in irgendeiner anderen Art erfolgen. So kann beispielsweise die Entleerung des zweiten Reservoirs 2 in einen Beutel erfolgen, oder in irgendeinen anderen Behälter oder Vorrichtung erfolgen.

## Patentansprüche

1. Absaugsystem für das Absaugen von Flüssigkeiten aus einem Operationsgebiet (9), mit einem Sauger (112), wobei der Sauger (112) über eine Absaugleitung (11) mit einem ersten Reservoir (1) verbunden ist, wobei das erste Reservoir (1) über eine erste Unterdruckleitung (121) mit einer ersten Unterdruckeinheit (12) verbunden ist, **dadurch gekennzeichnet, dass** das erste Reservoir (1) über eine Ventileinrichtung (16) mit einem zweiten Reservoir (2) verbunden ist, wobei das zweite Reservoir (2) über eine zweite Unterdruckleitung (221) mit einer zweiten Unterdruckeinheit (22) verbunden ist, und wobei mittels der Ventileinrichtung (16) die Verbindung zwischen dem ersten Reservoir (1) und dem zweiten Reservoir (2) geöffnet oder geschlossen werden kann.

2. Absaugsystem nach Anspruch 1, **dadurch gekennzeichnet, dass** das zweite Reservoir (2) mit einer weiteren Absaugleitung (211) verbunden ist, wobei die weitere Absaugleitung (211) zum Absaugen von Flüssigkeiten aus einem Operationsgebiet verwendbar ist.

3. Absaugsystem nach einem der Ansprüche 1 bis 2, **dadurch gekennzeichnet, dass** die erste Unterdruckeinheit (12) und/oder die zweite Unterdruckeinheit (22) mindestens eine Einstellvorrichtung (123,124,223,224) zur Einstellung eines selektierbaren Unterdrucks umfassen.

4. Absaugsystem nach einem der Ansprüche 1 bis 3, **dadurch gekennzeichnet, dass** das erste Reservoir (1) und/oder des zweite Reservoir (2) mindestens eine Filtriereinrichtung (14,24) zur Filtrierung von abgesaugten Flüssigkeiten umfassen.

5. Absaugsystem nach einem der Ansprüche 1 bis 4, **dadurch gekennzeichnet, dass** das zweite Reservoir (2) über eine Ablaufeinrichtung (26) mit einem Flüssigkeitsspeicher (8) verbunden ist.

6. Absaugsystem nach Anspruch (5), **dadurch gekennzeichnet, dass** der Flüssigkeitsspeicher (8) durch eine Herz-Lungen-Maschine gebildet wird.

7. Verfahren zum Absaugen von Flüssigkeiten aus einem Operationsgebiet (9) mit einem Absaugsystem nach einem der Ansprüche 1 bis 7, **dadurch gekennzeichnet, dass** die Ventileinrichtung (16) zwischen dem ersten Reservoir (1) und dem zweiten Reservoir (2) verschlossen wird und mittels der ersten Unterdruckeinheit (12) ein erster Unterdruck im ersten Reservoir (1) eingestellt wird, dass mittels der zweiten Unterdruckeinheit (22) ein zweiter Unterdruck im zweiten Reservoir (2) derart eingestellt wird, dass beim Öffnen der Ventileinrichtung (16) Flüssigkeit aus dem ersten Reservoir (1) in das zweite Reservoir (2) transportiert wird.
